Europäisches Patentamt

European Patent Office

Office européen des brevets

(11) Publication number: **0 384 566**
**A2**

## EUROPEAN PATENT APPLICATION

(21) Application number: 90300550.2

(51) Int. Cl.5: **C12Q 1/68, C07H 21/04**

(22) Date of filing: 18.01.90

(30) Priority: 27.01.89 US 302703

(43) Date of publication of application:
29.08.90 Bulletin 90/35

(84) Designated Contracting States:
**AT BE CH DE DK ES FR GB GR IT LI LU NL SE**

(71) Applicant: **THE WISTAR INSTITUTE**
**36th Street at Spruce**
**Philadelphia Pennsylvania 19104(US)**

(72) Inventor: **Reddy, E. Premkumar**
**547 Atterbury Road**
**Villanova, PA 19085(US)**
Inventor: **Koprowski, Hilary**
**334 Fairhill Road**
**Wynnewood, PA 19096(US)**

(74) Representative: **Hartley, David et al**
**Withers & Rogers 4 Dyer's Buildings Holborn**
**London, EC1N 2JT(GB)**

(54) Amplification of HTLV-1 sequences from multiple sclerosis patients.

(57) A method for diagnosing multiple sclerosis (MS) involves amplification of DNA sequences present in peripheral blood mononuclear cells of MS patients. The amplification is accomplished by use of the polymerase chain reaction employing primers derived from the sequence of HTLV-I, the aetiologic agent of adult T-cell leukemia. Particularly suitable primers are derived from the gag and env regions of the proviral genome.

# FIG. 1A

# FIG. 1B

# FIG. 1C

# FIG. 1D

# AMPLIFICATION OF HTLV-I SEQUENCES FROM MULTIPLE SCLEROSIS PATIENTS

## BACKGROUND OF THE INVENTION

Multiple sclerosis (MS) is a demyelinating disease of the central nervous system. Some features of the disease are shared by autoimmune diseases. For example, increases in absolute numbers of T-cells in the cerebrospinal fluid (CSF) and disturbances in the relative proportions of T-cell sub-populations during exacerbation or remission of the disease are characteristic of both MS and autoimmune diseases. A high level of oligoclonal immunoglobulin in CSF of MS patients is often found.

Further, there are common characteristics shared by autoimmune diseases and human retrovirus infections. The human T-cell is the main target of all known human retroviruses. T4-positive (helper-inducer) lymphocytes are transformed by human T-cell lymphotropic virus HTLV-I and -II and are killed by HIV-I, resulting in T-cell sub-population imbalances. Occasionally, polyclonal B-cell activation and immunoglobulin production has been associated with human retrovirus infections.

Previously, antibodies have been found in both serum and cerebrospinal fluid (CSF) of patients with MS which are partially cross-reactive with the HTLV-I gag (p24) protein. (Koprowski et al., Nature, vol. 318, p. 154, 1985.) In addition, it was reported that about one-third of the patients also had nucleic acid in lymphocytes of the CSF which hybridized to HTLV-I LTR sequences (long terminal repeat) as well as to gag p24 coding sequences. (Koprowski, supra.) However, the number of lymphocytes which had hybridizing nucleic acids was very low, less than about $10^{-4}$, and these were only detected under conditions of low stringency.

Ohta, et al. (Journal of Immunology, vol. 137, pp. 3440-43, 1986) confirmed the relationship of an HTLV-I-like virus to MS. They reported that 24% of MS patient sera tested contained antibodies which detected HTLV-I gag proteins (p15, p19, p24) on Western blots. The MS sera did not react with the precursor protein (p53) nor with env protein (p46).

While these findings strongly implicate a virus related to HTLV-I as being associated with MS, there is a need in the art for a more reliable and sensitive assay to detect the virus in patient specimens.

## SUMMARY OF THE INVENTION

It is an object of the invention to provide a method of diagnosing multiple sclerosis.

It is also an object of the invention to provide primers useful for diagnosing multiple sclerosis.

These and other objects of the invention are provided by one or more of the embodiments described below.

In one embodiment a method is provided for diagnosing a human suffering from multiple sclerosis. Peripheral blood mononuclear cells are collected from a human displaying symptoms of a neurological disease. The cellular DNA is extracted from the mononuclear cells and used as a template for repeated cycles of DNA synthesis to amplify portions of the cellular DNA. Primers for the DNA synthesis are pairs of oligonucleotides whose sequences correspond to the HTLV-I proviral genome. The primers prime on opposite strands of the cellular DNA such that amplified DNA sequences form a duplex of less than about 3 kb. Thus, the primers correspond to sequences of the HTLV-I proviral genome which are less than about 3 kb apart. The presence of the amplified DNA duplex is detected and the presence of said duplex is related to multiple sclerosis.

In another embodiment of the invention primers are provided which are derived from HTLV-I and which are useful for diagnosing multiple sclerosis. The primers comprise at least about 22 nucleotides and have a G/C base content of at least about 50%. The primers are capable of priming DNA synthesis on a DNA template comprising DNA extracted from peripheral blood mononuclear cells of an MS patient. In preferred embodiments the primers are derived from the gag region or the env region of the HTLV-I genome. In another preferred embodiment at least one restriction enzyme site is present in each primer.

The present invention provides the art with a sensitive and reliable assay to determine infection by an HTLV-I-like virus in MS patients. All patients so far tested who have been diagnosed as having MS are detected by the assay of the present invention. This contrasts with only about 40% of the MS patients detected by prior art methods.

## BRIEF DESCRIPTION OF THE DRAWINGS

Figure 1 shows gene amplification analysis of cells known to be positive or negative for HTLV-I using primers from the HTLV-I gag (panels A and B) and env (panels C and D) genes. Panels A and C show ethidium bromide staining and panels B and D show hybridization to labeled oligonucleotide probes.

Figure 2 shows gene amplification analysis of MS patients using primers from HTLV-I gag (panel A) and env genes (panel B). Hybridizations with labeled oligonucleotide probes are shown.

Figure 3 shows restriction enzyme cleavage analysis of plasmid clones derived from amplified DNA duplexes from MS patients. DNA in even numbered lanes was cleaved with Pst I and Sma I, and that in odd numbered lanes was cleaned with Pst I, Sma I and Nco I. Lanes marked M contain molecular weight markers, i.e., phi-X DNA digested with Hae III.

Figure 4 provides the nucleotide sequence of two amplified and molecularly cloned sequences from the gag region (panel A) of two MS patients and the env region (panel B) of one MS patient. These were compared to the sequence reported by Seiki et al., P.N.A.S. (USA) vol. 80, p. 3618, 1983, for HTLV-I, denoted ATK-1.

Figure 5 shows DNA amplification analysis with gag primers comparing adherent and non-adherent cells as sources of amplifiable DNA.

## DETAILED DESCRIPTION

It is a discovery of the present invention that primers derived from the sequence of the HTLV-I proviral genome can be used to prime DNA synthesis on DNA templates isolated from peripheral blood mononuclear cells of MS patients. The products of such primed DNA synthesis can be detected by a number of techniques known in the art, and the presence of specific DNA products is related to MS. Such specific DNA products were detected in 6/6 MS patients but only in 1/20 asymptomatic donors, presumed to be disease-free.

Patients who would generally be diagnosed using such a method are those displaying symptoms of neurological disease. Specifically, those patients who are suspected of having MS would be candidates for such a procedure. The accepted criteria for a diagnosis of MS are those set forth by McDonald and Halliday, (British Medical Bulletin, vol. 33, p. 4, 1977). These include: lesions at two or more distinct sites in the white matter of the central nervous system, oligoclonal IgG in the CSF, optic neuritis, progressive spastic paraplegia, diplopia, vertigo.

Peripheral blood mononuclear cells (PBMC) are isolated from the peripheral blood of patients and healthy donors according to methods well known in the art. One method of obtaining such cells is described in A. Boyum, J. Clin. Lab. Invest., vol. 21 (suppl. 97) p. 1, 1968. This method involves fractionation on Ficoll/Hipaque gradients. Other methods may of course be used. Mononuclear cells thus isolated generally include monocytes, macrophages and lymphocytes.

Once the mononuclear cells have been isolated from the peripheral blood, genomic DNA, called here cellular DNA, is extracted. One particular method of cell lysis and DNA extraction is described in Maniatis, et al. Molecular Cloning, Cold Spring Harbor Laboratory, 1982, at page 280. This method involves cell lysis with detergent and proteinase, followed by phenol extraction, dialysis, and RNase treatment. Other methods may be used to attain cellular DNA preparations. Desirably, the average size of the DNA fragments is greater than about 0.5 kb.

In order to amplify the cellular DNA, the polymerase chain reaction is used. This technique has been described inter alia, in U.S. Patents 4,683,202, and 4,683,195, Saiki et al, Science, vol. 230, p. 1350, 1985; Mullis et al. Meth. Enzymol., vol. 155, p. 355, 1987; Saiki et al., Nature, vol. 324, p. 163, 1986; Scharf et al., Science, vol. 233, p. 1076, 1986. Briefly, a molar excess of primers and cellular DNA template are denatured and annealed and mixed with all four deoxyribonucleotide triphosphates as well as a DNA polymerase, preferably Taq polymerase which is a thermostable enzyme. For each cycle of DNA synthesis, the reaction mixture is denatured, e.g. at 94°C for about 2 minutes, and annealed, e.g., for about 1 minute at 55°C. The DNA synthesis is done at about 70°C in the case of catalysis by Taq polymerase for about 2 minutes. Fresh enzyme can be added to the reaction mixture after about every ten cycles. Approximately 36 to 40 cycles are generally sufficient for detection of amplified DNA duplexes in MS patient DNA samples. Each cycle consists of denaturation of DNA duplexes, annealing of DNA duplexes, and primer extension (DNA synthesis).

Priming DNA synthesis, according to the present invention, refers to the DNA polymerase-catalyzed synthesis of DNA by extension of a primer which has been annealed to a larger DNA template. The DNA which is synthesized as an extension of the primer is complementary to the sequence of the DNA template. DNA templates, in the method of the invention comprise high molecular weight genomic DNA (cellular DNA) isolated from PBMC of humans, either healthy donors or neurological disease patients.

The paired primers which are used to amplify discrete segments of amplified DNA duplex are derived from the sequence of the HTLV-I proviral genome. They may be synthetic oligonucleotides or isolated from biological material. Typically they are chosen so that each primer contains a restriction enzyme site. This is to facilitate cloning of the amplified DNA duplex. Alternatively, a restriction site can be introduced into a primer, preferably at the 5′ end, so that it does not interfere with primer extension to the 3′ end. Desirably, the primers correspond to the sequence of the HTLV-I proviral genome exactly, except where restriction sites have been introduced. (The proviral genome is the viral DNA as it exists in integrated form in a host cell.) The primers of a primer pair each hybridize to opposite strands of the same DNA template. Usually they are separated by no more than about 3 kb of the template. Above this distance, the amplification of intervening DNA is not efficient. The result of the repeated cycles of DNA synthesis is an amplified DNA duplex having at each end the sequence of one of the primers of the primer pair as part of the duplex.

Primers of the present invention are longer than about 22 nucleotides and contain greater than about 50% of the base composition as guanine and cytosine (G/C). The primers are capable of priming DNA synthesis on a DNA template comprising DNA extracted from PBMC of an MS patient. Preferred primers of the present invention are from the gag and env genes of the HTLV-I proviral genome. Particular primer pairs which have been shown to be efficient at detecting HTLV-I-related sequences in MS patient DNA samples are 5′-CGACCGCCCGGGGGCTGGCCGCT-3′, and 5′-GGTACTGCAGGAGGTCTTGGAGG-3′; 5′-CTCCCTTCTAGTCGACCTCCAGG-3′ and 5′-GCCACCGGTACCGCTCGGCGGGAG-3′.

To find other primers useful in the diagnosis of MS a simple test can be performed. The primers whose sequences are disclosed here can be used as a positive control to identify an MS patient with amplifiable HTLV-I-related DNA sequences. Such a patient can be used as a source of PBMC DNA to use as template to test other primer candidates. If the primer candidates are able to prime DNA synthesis leading to a discrete DNA duplex which is detectable by any of the means discussed herein, that primer candidate is useful for the diagnosis of MS.

Detection of a DNA duplex can be performed by any means known in the art. The presence of the DNA duplex is an indication that the patient has multiple sclerosis. In some cases the DNA synthesis reaction mixture can be electrophoretically separated and the DNA duplex visualized as a discrete band merely by ethidium bromide staining. Typically such electrophoretic separation is done on agarose or polyacrylamide gel matrices. To confirm the identify of the discrete band, restriction enzymes can be used which cleave within the amplified segment, and the products of such cleavage can be visualized. However, in the case of MS mononuclear cells, the level of infected cells is apparently very low. Therefore, sometimes direct visualization of amplified bands is not a sensitive enough detection means.

To detect amplified DNA duplexes more sensitively, labeled oligonucleotide probes can be hybridized to the amplified DNA duplex, e.g., by the Southern transfer technique. This technique is well known in the art. See, e.g., Maniatis, supra, at page 382. The oligonucleotide probe can be labeled by nick translation or end labeled with a radioactive DNA component. Again, such labeling techniques are well known. The oligonucleotide probe should be selected to span the region of the DNA duplex. That is, its sequence should hybridize with sequences known to be between the two primers on the HTLV-I proviral genome.

An even more sensitive means of detecting the amplified DNA duplex is by molecular cloning. If the primers were designed to contain restriction enzyme sites, then the DNA duplex can be cleaved with those enzymes to create joining sites to a vector. Alternatively, short DNA linkers containing restriction enzyme sites can be ligated to the ends of the DNA duplex for subsequent cleavage and ligation to an appropriate vector. Other molecular cloning schemes are possible and may be used. It is desirable to use the most efficient cloning scheme to maximize sensitivity of detection.

Any cloning vector may be used which is known in the art, e.g. Bluescript (Stratagene). Once a vector is ligated to the amplified DNA duplex, bacteria can be transformed with the ligation mixture to form bacteria which carry the molecular clones of the amplified DNA duplex. The transformed bacteria are plated out to form colonies and the colonies are screened for hybridization of their cellular DNA to oligonucleotide probes, such as those described above. The presence of hybridizing colonies indicates that the mononuclear cell DNA which was used as a template for DNA synthesis contains HTLV-I related sequences. Detection of the DNA duplex by any of the means discussed above or any other means known in the art indicates that the patient has multiple sclerosis.

The following examples are not intended to limit the scope of the invention which is defined by the claims appended below as well as the foregoing discussion.

Example 1

4

This example describes the patients used for both MS and control purposes.

The MS patients studied consisted of three males and three females with MS as defined according to McDonald and Halliday (Br. Med. Bull., vol. 33, p. 4, 1977). The patients were all from Sweden and were diagnosed and treated at the Department of Neurology, University Hospital, University of Lund, Sweden. Table 1 below shows some of the individual characteristics of the patients.

Table 1

| Patient code | Age/sex | Duration of disease (years) | Initial symptoms | Clinical status at sampling + | HTLV-I* antibody | HTLV-I sequences in mononuclear cells | | PCR | |
|---|---|---|---|---|---|---|---|---|---|
| | | | | | | PCR/DNA | Molecular cloning sequence analysis | Adherent cell DNA | Nonadherent cell DNA |
| 01-12 | 44/M | 10 | Sensory | Remission | + | + | + | + + + + | + /- |
| 13-10 | 23/M | 12 | Diplopia | Remission | - | + /- | + | + + + + | + /- |
| 10-02 | 21/M | 3 | Sensory | Remission | ? | + | + | | |
| 01-14 | 29/F | 8 | Diplopia | Exacerb. | + | + /- | + | + + + | + /- |
| 09-1920 | 36/M | 12 | Optic neutis | Progres. | - | + | + | | |
| 05-08 | 23/F | 2 | Useless hand | Exacerb. | - | + | + | + + + + | - |
| Controls | | | | | - | 1/20 | - | 0/13 | 1/13** |

*Binding to disrupted HTLV-I virons.

+ All patients and had undergone exacerbation and remissions since the onset of disease; patient 09-1920 has had chronic progressive disease for the last 4 years. Patient 05-08 was treated with corticosteroids and ACTH; none of the o her patients or controls had been treated.

EP 0 384 566 A2

All patients had a moderate mononuclear pleocytosis in CSF and five had oligoclonal immunoglobulin on isoelectrofocusing. The patient with no oligoclonal bands had multiple bilateral lesions on magnetic resonance imaging compatible with MS. At the time of the study, three patients had exacerbrations and one of these was treated with corticosteroids. None of the patients had ever received a blood transfusion. Negative controls were ten healthy subjects from Sweden who had no antibodies for HTLV-I and ten healthy blood donors from Philadelphia.

Results of ELISA assays with the use of disrupted HTLV-I virions performed as described in Koprowski, supra, revealed the presence of antibodies in serum samples from two of the six patients at the time their PBMC were tested for the presence of HTLV-I sequences (Table 1).

Example 2

This example describes the techniques and materials used to amplify HTLV-I sequences in MS patients.

Blood samples were obtained from the cubital vein of both healthy and MS donors, and the peripheral blood mononuclear cells (PBMC) were processed according to Boyum, J. Clin. Lab. Invest., vol. 21 (suppl. 97), p. 1, 1968. Cellular DNA was extracted from the PBMC according to methods well known in the art.

To amplify HTLV-I sequences in the cellular DNA extracted from PBMC, we used primer pairs from the gag and env region. The two gag primers were 5'-CGACCGCCCCGGGGGCTGGCCGCT-3 and 5'-GGTACTGCAGGAGGTCTTGGAGG-3. These primers would be expected to amplify the region between nucleotides 842 and 1376 of the sequence described by Seiki et al. (Proc. Natl. Acad. Sci. U.S.A. 80, 3618 (1983). The two env primers were 5'-CTCCCTTCTAGTCGACGCTCCAGG-3 and 5'-GCCACCGGTACCGCTCGGCGGGAG-3. These primers would be expected to amplify the region between nucleotides 5684 and 6151 of Seiki et al. These primers were greater than 22 bases long and rich in G-C content to allow stable hybridization. The gag primers included the cleavage sites for Sma I and Pst I and the env primers contained the cleavage sites for Sal I and Kpn I, so that after amplification the DNA fragments could be cleaved with these restriction enzymes and cloned into a plasmid vector.

Amplification of the DNA was performed with the Geneamp kit provided by Perkin-Elmer Cetus Corp. (Norwalk, CT). The reactions were carried out under the conditions specified by the manufacturer with 2 ug of DNA and 1.0 umol of the primers. The reaction mixtures contained 25 mM TAPS-Cl pH 9.3; 50 mM KCl, 2 mM $MgCl_2$, 1 mM DTT, 200 uM each of dATP, dGTP, dTTP, and dCTP in a final volume of 50 $\mu$l. 2.5 units of Taq polymerase was used for each assay. Typically, for each cycle of amplification, the mixture was denatured at 94°C for 2 min., annealed at 55°C for 1 min., and then extended at 70°C for 2 min. From 36 to 40 cycles of amplification were performed and fresh enzyme (2 to 5 U) was added to each tube at the end of every tenth cycle.

Example 3

This example describes a method of detecting amplified sequences using hybridzation to an oligonucleotide probe.

In one method used to detect amplified sequences, we used either a nick-translated probe derived from the HTLV-1 proviral genome that spanned the amplified regions or synthetic oligonucleotide probes that were labeled at the 5' end with $^{32}PO_4$ and that were complementary to the sequences that were amplified. For example, the oligonucleotide probe for the amplified gag DNA was 5'-GATCCCGTCCCGTCCCGCGCCA-3', which spans the region between nucleotide 1081 and 1102 of the published HTLV-I sequence. The oligonucleotide probe for the env region was 5'-GCCTCTCCACTTGGCACGTCC-3' spanning nucleotide 5899 to 5919.

The region of amplification for gag region contained a unique Nco I site, while the amplified env region contained a Cla I site for further diagnostic purposes. Thus, amplification of HTLV-I sequences with gag and env primers was expected to yield sequences of 530 and 467 bp, respectively. The amplified gag sequences, when cleaved with Nco I, would yield two fragments of 390 and 140 bp long, while the cleavage of env sequences with Cla I would yield fragments of 263 and 204 bp.

Example 4

This example describes test procedures used to test the primers, probes and techniques described above in Examples 2 and 3.

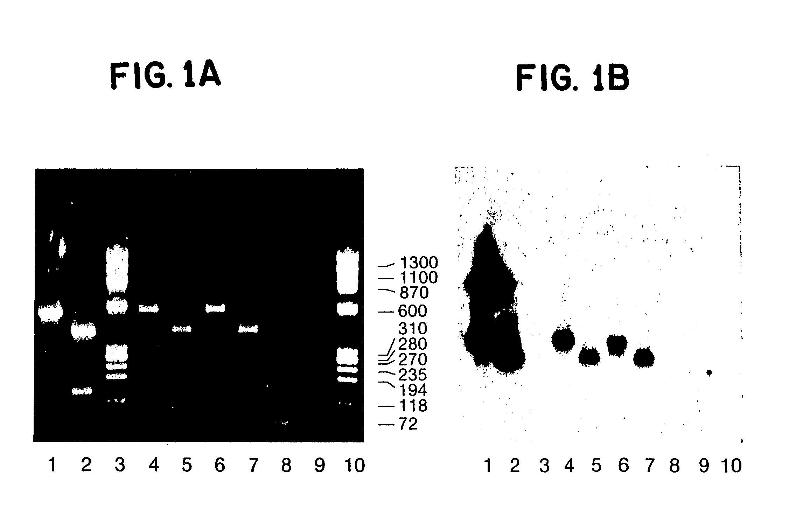

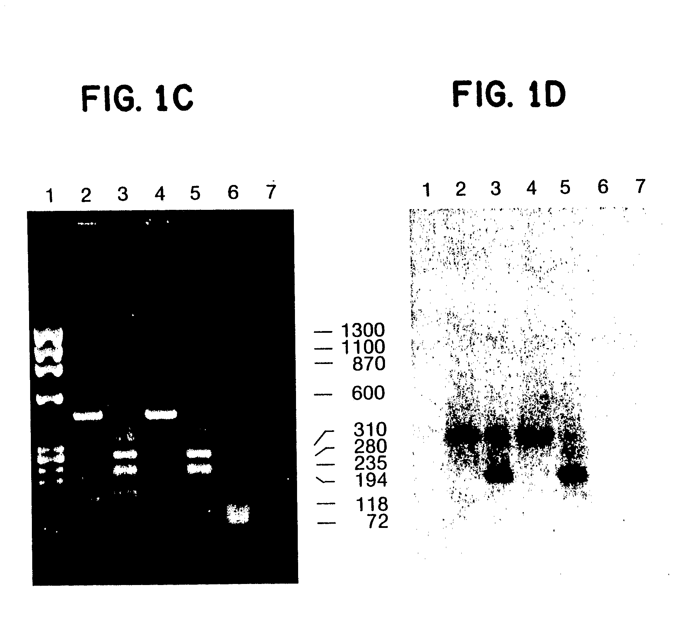

We tested our strategy with four DNA samples: (i) a plasmid DNA containing the HTLV-I proviral genome; (ii) total cell DNA isolated from the MT-2 cell line, which is infected with HTLV-1 (Yoshida, et al., Proc. Natl. Acad. Sci. U.S.A. 79,2031 (1982)); (iii) cell DNA derived from PBMC of a patient with chronic encephalomyelopathy initially diagnosed as tropical spastic paraparesis, or TSP (Jacobson, Nature 331, 540 (1988); Reddy et al., Proc. Natl. Acad. Sci. U.S.A. 85, 3599 (1988)); and (iv) normal cell DNA (See Figure 1). Distinct amplification of gag and env sequences occurred in the three cell DNAs that contained the HTLV-I proviral genome (see Fig. 1A, lanes 1,4 and 6 and Fig. 1C, lanes 2 and 4). The band with the highest intensity was from the plasmid DNA that contained the cloned proviral genome; that with the least intensity was from the "TSP" patient DNA.

No nonspecific amplification of DNA sequences occurred when the primers were used in combination with normal cell DNA (Fig. 1A, lanes 8 and 9, and Fig. 1C, lanes 6 and 7). When the amplified gag sequence were cleaved with Nco I, all three samples yielded two restriction fragments of 390 bp and 140 bp, further demonstrating the specificity of amplification (Fig. 1A, lanes 2,5 and 7). Similar results were obtained with env sequences where cleavage with Cla I showed two bands of 260 and 200 bp long (Fig. 1C, lanes 3 and 5). Blot hybridization with gag- and env-specific oligonucleotide probes (described in Example 2) revealed hybridization to the amplified sequences (Fig. 1B, lanes 1, 2, 4-7, and 1D, lanes 2-5). These results showed that amplification of the proviral sequences could be achieved by using the primers we had chosen, that this amplification was specific for HTLV-1 sequences, and that no amplification of nonspecific or endogenous viral sequences occurred under the conditions used.

Each of the lanes in Figure 1 is explained as follows: DNAs isolated from bacterial plasmid (A and B, lanes 1 and 2); MT-2 cell line infected with HTLV-I (A and B, lanes 4 and 5; C and D, lanes 2 and 3); peripheral blood lymphocytes of a TSP patient 3-19-3 (A and B, lanes 6 and 7; C and D, lanes 4 and 5); and a human cell line ML-2 known to be negative for HTLV-1 sequences (A and B, lanes 8 and 9; C and D, lanes 6 and 7). Lanes 3 and 10 (A and B) and lane 1 (C and D) contain molecular weight markers (phi-X DNA digested with Hae III). In panels A and B, lanes 1,4,6,8 contain undigested DNA while lanes 2,5,7 and 9 contain DNAs digested with Nco I. In panels C and D, lanes 2, 4, 6 contain undigested DNAs while lanes 3, 5, 7 contain DNAs digested with Cla I.

Example 5

This example describes the use of the primers of the present invention to amplify sequences from MS patient PBMC DNA, and the detection of amplified sequences by ethidium bromide staining or Southern hybridization.

DNA samples derived from PBMC and peripheral blood lymphocytes of 26 individuals were tested: the six patients with MS (Table 1) and 20 controls. Amplification with gag primers of HTLV-I sequences occurred in all six samples from MS patients, and the amplified 530-bp DNA fragment was visually detectable upon electrophoretic separation and staining with ethidium bromide. The intensity of the amplified DNA band was much lower than that of the positive controls from MT-2 cells and amplified DNA from cells of the patient with TSP.

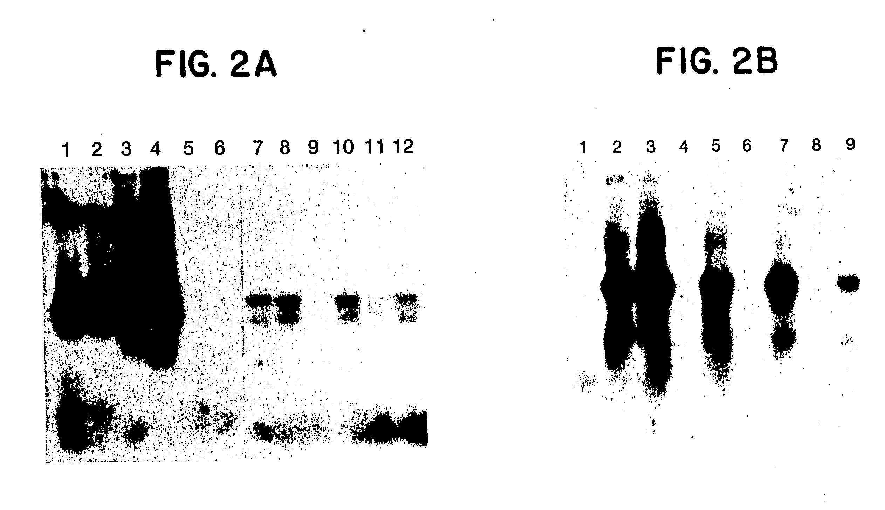

In general, analysis of samples from all patients showed bands visualized readily after ethidium bromide staining and hybridization with the probe. However, in some experiments (two out of four) these bands could not be visualized readily and the hybridization intensity was very low, making it virtually undetectable (see Fig. 2A, lanes 9 and 11). Similar results were obtained with the amplified env sequences (Fig. 2B) where, in two out of four experiments, we could clearly see an amplified band that could be hybridized to the envelope-specific probes (Fig. 2B, lanes 5, 7, and 9), whereas in the other two experiments such clear amplification could not be detected by amplification and DNA hybridation(Fig. 2B, lanes 4, 6, and 8).

Of the ten amplified control DNA samples from individuals of Swedish origin, one contained detectable HTLV-I sequences. The other nine samples were negative. All ten amplified control DNAs from the American population were negative. The appearance of HTLV-I-related sequences in a normal individual with no signs of leukemia or MS was unexpected and is being further investigated.

Figure 2, panel A, shows in lane 1, MT-2 cell line infected with HTLV-1; lane 2, peripheral blood lymphocytes of the TSP patient 3-19-3; lane 3 plasmid containing proviral genome of HTLV-I; lane 4, plasmid containing HTLV-I proviral genome isolated from the TSP patient 3-19-3; lane 5, ML-2 cells free of retroviral infection; lane 6, peripheral blood cells of a normal individual; and lanes 7-12, peripheral blood cells derived from the six MS patients under study. Lane 7, patient 09-1920; lane 8, patient 01-12; lane 9, patient 01-14; lane 10, patient, 05-08; lane 11, patient 13-10; and lane 12, patient 10-02. Figure 2, panel B, shows DNA in lane 1, from ML-2 cell line; lane 2, MT-2 cell line; lane 3, peripheral lymphocytes of TSP patient 3-19-3; lane 4, patient 09-1920; lane 5, patient 01-12; lane 6, patient 01-14; lane 7 patient 05-08; lane 8, patient 13-10; and lane 9, patient 10-02. The DNAs were used in amplification analysis, electrophoretically separated on 2% agarose gels, transferred to a nitrocellulose membrane, and hybridized with a $^{32}$P-labeled oligonucleotide probe.

## Example 6

This example demonstrates the use of molecular cloning to detect amplified products from MS patient DNA.

The difficulty of detection of amplified sequences from MS patients by ethidium bromide staining and Southern hybridization was circumvented as shown below by molecular cloning of the amplified sequences.

The DNAs from MS patients were amplified with the two pairs of primers described above, cleaved with the appropriate restriction enzymes (Pst I and Xma I for gag sequences, and Sal I and Kpn I for env sequences), and ligated to Bluescript vector DNA (Stratagene) that was also cleaved with Xma I and Pst I or Sal I and Kpn I. Bacteria were transformed with the ligated DNA and positive clones were selected by hybridization with a nick-translated probe of HTLV-1 proviral DNA or a labeled oligonucleotide probe (described above in Example 2). All six MS patient DNA samples from all experiments had several positive clones whether amplification was seen by the DNA blotting technique or not. No positive clones were observed from control DNA ligations.

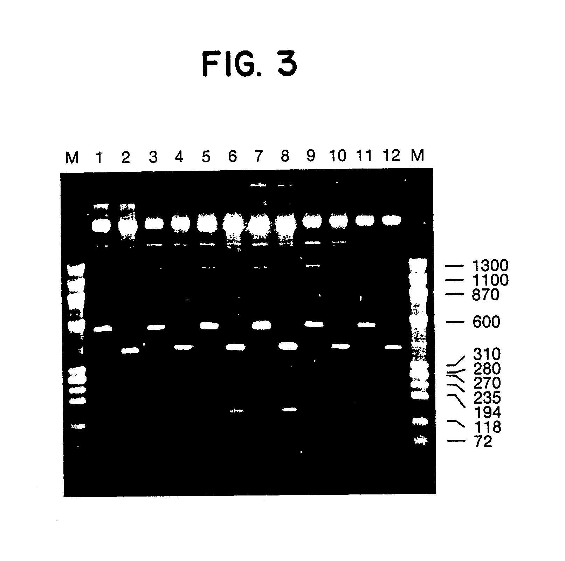

Four to six positive colonies from each sample were colony-purified and the plasmid DNA was isolated. Restriction enzyme analysis of the purified DNA (Fig. 3) showed that each gag clone released an insert of 530 bp when cleaved with Pst I and Xma I; this fragment could be further cleaved with Nco I into two fragments of 390 and 140 bp. Similarly, all envelope clones released an insert of 460 bp with Sal I and Kpn I, which could be further cleaved with Cla I into two fragments of 260 and 200 bp.

Figure 3 shows DNA in lanes 1 and 2, from clones from DNA of patient 09-1920; lanes 3 and 4, clones from DNA of patient 01-12; lanes 5 and 6, clones from DNA of 01-14; lanes 7 and 8, clones from DNA of patient 05-08; lanes 9 and 10 clones from DNA of patient 13-10; and lanes 11 and 12, clones from DNA of patient 10-02. Lanes 1, 3, 5, 7, 9 and 11 contain plasmid DNA cleaved with Pst I and Sma I while lanes 2, 4, 6, 8 and 10 contain the same DNAs cleaved with Pst I, Sma I and Nco I. Lanes marked M contain molecular weight markers (phi-X DNA digested with Hae III).

## Example 7

This example describes the sequence analysis of the molecularly cloned, amplified sequences from MS patients.

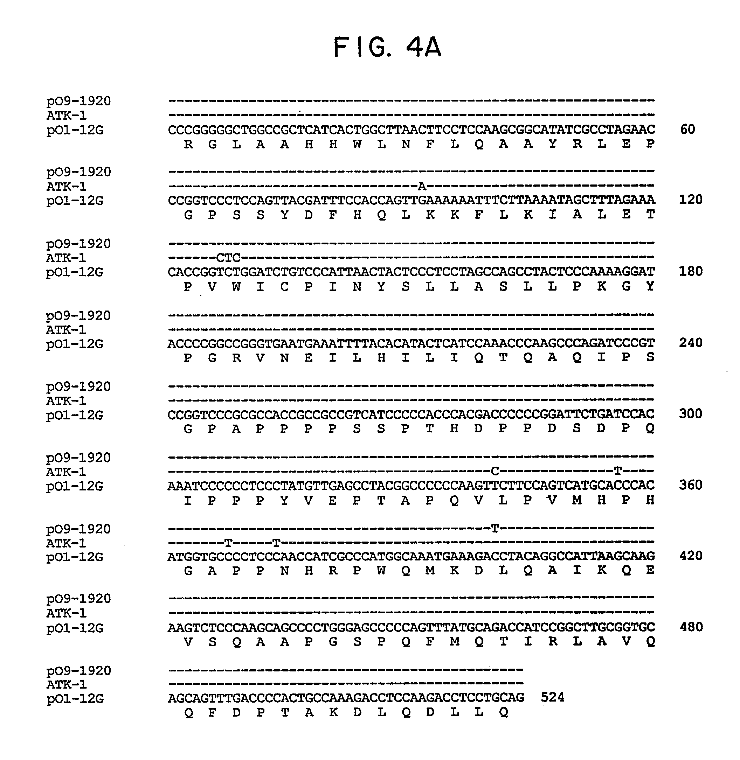

We analyzed the sequence of the gag and env inserts using a combination of the methods of Sanger et al. (Proc. Natl. Acad. Sci U.S.A. 74, 5463 (1977)) and Maxam and Gilbert (Methods Enzymol. 65. 499(1980)). Five of the clones derived from the MS patient DNAs had identical gag sequences while a sixth clone (p09-1920) differed in one position from the others (Fig. 4A). All six clones differed from the published sequence of a Japanese isolate (ATK-1, see Seiki et al.. supra) at six positions. The sequence of the HTLV-I clone derived from the MT-2 cell line (Yoshida et al., P.N.A.S. (USA) vol. 79, p. 203, 1982) was identical to clone p01-12.

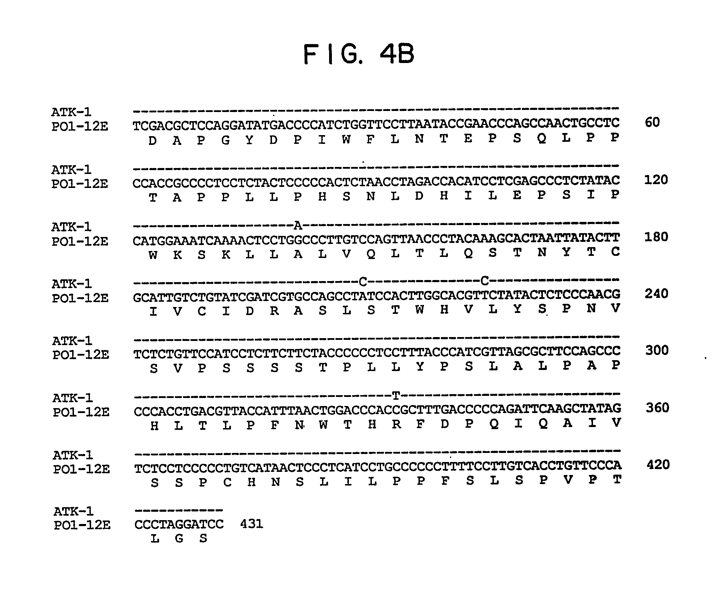

The env inserts (Fig. 4B) contained at least two point mutations that differed from the ATK-1 sequence as well as from the clone from the MT-2 cell line. These mutations suggest that the HTLV-I provirus in the six MS patients, though similar in many respects to the classical HTLV-I clone, differs at least in some positions in the envelope region.

Figures 4A shows the nucleotide sequence analysis of the Sma I-Pst I gag fragment cloned from the amplified DNA of one of the MS patients (01-12). The sequence differences between this clone and that of ATK-I are indicated on top of the sequence; the deduced amino acid sequence is given below. The sequence of clones from four other patients (13-10, 10-02, 01-14, 05-08) was identical to this sequence. The sequence of the clone from patient 09-1920 differed in one position which is indicated.

Figure 4B shows the nucleotide sequence analysis of the Sal I and Kpn I env fragment cloned from the amplified DNA of one of the MS patients (01-12). The sequence differences between this clone and ATK-1 are indicated. The sequence of clones derived from five other patients (13-10, 10-02, 01-14, 05-08, 09-1920) was identical to this sequence.

Example 8

This example demonstrates that HTLV-I related sequences are associated predominantly with adherent cell DNAs.

Studies of HIV-I-infected individuals indicate that monocyte/macrophage cell populations are targets for virus infection and in some patients these infected cells occur in brain tissue. To test whether the HTLV-I related sequences in MS patients were present in lymphocytes or nonlymphocytic cells, we cultured PBMC from four of the MS patients and 13 healthy subjects in tissue culture flasks for 18 to 72 hours, and separated the adherent and non-adherent populations.

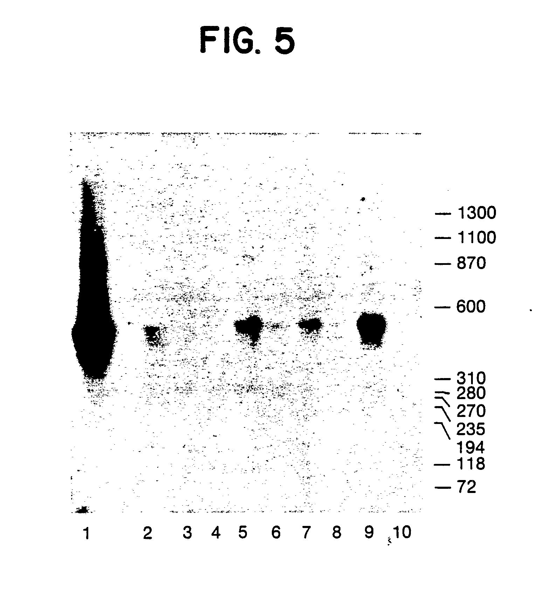

When the DNA from these cell populations was amplified, the HTLV-I related sequences were associated predominantly with adherent cell DNAs (Fig. 5, lanes 5, 7 and 9) in samples from the MS patients; non-adherent cell DNAs from the patients also contained low levels of hybridizable sequences (Fig. 5, lanes 6 and 8). When amplified sequences were cloned, both adherent and non-adherent cells were found to be positive. In contrast, the DNAs from the 13 normal subjects showed no hybridizing sequences under similar conditions (Fig. 5, lanes 3 and 4). HTLV-I-related sequences in cells of the one healthy subject from Sweden were found only in the non-adherent lymphocyte population.

Figure 5 shows in lane 1, plasmid containing HTLV-I proviral genome; lane 2, MT-2 cells infected with HTLV-I; lane 3, adherent cells from a healthy individual; lane 5, non-adherent cells from a healthy individual; lanes 5, 7, and 9, adherent cells from peripheral blood cells of MS patients 01-12, 01-14, and 05-08, respectively; and lanes 6, 8, and 10, non-adherent cells derived from the same MS patients as in lanes 5, 7 and 9.

Example 9

This example describes the testing and evaluation of primers for amplification of HTLV-I-related sequences in MS patients.

The primers described above in Example 2, was successful in detecting even the low level of HTLV-I related sequences found in MS patient mononuclear cells. In general, these primers are greater than about 22 nucleotides in length and have a G/C base content of greater than about 50%. It is also desirable that the 5′ and 3′ ends of the primer not be complementary so that the primer does not self-hybridize rather than hybridizing with the template DNA.

In order to determine empirically which primers are useful in diagnosing MS they must be tested for priming ability on MS patient mononuclear cells, as described above in Example 2. As molecular cloning is the most sensitive detection means of amplified DNA known, techniques such as that described in Example 6 should be used to detect the amplification.

The following primers were tested for priming and amplification of MS patient DNA. Although they are derived from HTLV-I gag and env genes, they did not yield positive results with MS DNA. They were however able to prime detectable amplification from a TSP patient. Primer pair #1 for gag gene: 5′-CGCGAGCTCTATTCCGCGAC-3′ and 5′-CCGAGCCGGTGTTTCTAGAG-3′. Primer pair #2 for gag gene: 5′-CGACCGCCCCGGGGGGCT-3′ and 5′-GGTACTGCAGGAGGTCT-3. Primer pair #1 for env gene: 5′-CTCTCGAGTTGGCACGTCCT-3′ and 5′-CCTCGAGTGGGAACAGGTGAC-3′. Primer pair #2 for env gene: 5′-CTAGTCGACGCTCCAG-3′ and 5′-GCCACCGGTACCGCT-3′.

**Claims**

1. A method of diagnosing a human suffering from multiple sclerosis comprising:
collecting peripheral blood mononuclear cells from a human displaying symptoms of a neurological disease;
extracting cellular DNA from the mononuclear cells;
amplifying portions of the cellular DNA by repeated cycles of priming DNA synthesis on the cellular DNA as template using paired DNA primers corresponding to sequences of the HTLV-I proviral genome, said primers priming on opposite strands of the template, to form an amplified DNA duplex of less than about 3 kb;
detecting the presence of the DNA duplex, and relating the presence of said duplex to multiple sclerosis.

2. The method of claim 1 wherein the peripheral blood mononuclear cells are selected from the group consisting of monocytes and macrophages.

3. The method of claim 1 wherein the peripheral blood mononuclear cells are lymphocytes.

4. The method of claim 1 wherein the DNA duplex is detected by hybridization to a labeled oligonucleotide probe, the sequence of said probe corresponding to the HTLV-I proviral genome and spanning the region corresponding to the amplified DNA duplex.

5. The method of claim 1 wherein the amplified DNA duplex is detected by molecular cloning of said DNA duplex in bacteria and hybridizing the cellular DNA of the bacteria containing the molecular clone to a labeled oligonucleotide probe, the sequence of said probe corresponding to the HTLV-I proviral genome and spanning the region corresponding to the amplified duplex DNA.

6. The method of claim 1 wherein the amplified DNA duplex is detected by visualization on an electrophoretic gel matrix.

7. The method of claim 1 wherein the paired DNA primers span the gag region.

8. The method of claim 1 wherein the paired DNA primers span the env region.

9. The method of claim 1 wherein the paired DNA primers are at least about 22 nucleotides in length, have a G/C base content of at least about 50%, and are capable of priming DNA synthesis on a DNA template comprising DNA extracted from peripheral blood mononuclear cells of an MS patient.

10. The method of claim 7 wherein the two gag region primers are 5'-CGACCGCCCCGGGGGCTGGCCGCT-3' and 5'-GGTACTGCAGGAGGTCTTGGAGG-3'.

11. The method of claim 8 wherein the two env region primers are 5'-CTCCCTTCTAGTCGACGCTCCAGG-3' and 5'-GCCACCGGTACCGCTCGGCGGGAG-3'.

12. An HTLV-I-derived primer useful for diagnosing multiple sclerosis, said primer comprising at least about 22 nucleotides, having a G/C base content of at least about 50%, having a sequence corresponding to the HTLV-I proviral genome, and capable of priming DNA synthesis on a DNA template comprising DNA extracted from peripheral blood mononuclear cells of an MS patient.

13. The primer of claim 12 which is derived from the gag region.

14. The primer of claim 12 which is derived from the env region.

15. The primer of claim 13 which is 5'-CGACCGCCCCGGGGGCTGGCCGCT-3'.

16. The primer of claim 13 which is 5'-GGTACTGCAGGAGGTCTTGGAGG-3'.

17. The primer of claim 14 which is 5'-CTCCCTTCTAGTCGACGCTCCAGG-3'.

18. The primer of claim 14 which is 5'-GCCACCGGTACCGCTCGGCGGGAG-3'.

19. The primer of claim 12 wherein the primer contains a site for a restriction enzyme.

# FIG. 1A

# FIG. 1B

| | | | | | | | | | |
|---|---|---|---|---|---|---|---|---|---|
| | | | | | | | | | ⎼ 1300 |
| | | | | | | | | | ⎓ 1100 |
| | | | | | | | | | ⏜ 870 |
| | | | | | | | | | ─ 600 |
| | | | | | | | | | 310 |
| | | | | | | | | | ⧸ 280 |
| | | | | | | | | | ⫤ 270 |
| | | | | | | | | | ⏜ 235 |
| | | | | | | | | | ⏜ 194 |
| | | | | | | | | | ─ 118 |
| | | | | | | | | | ─ 72 |

1  2  3  4  5  6  7  8  9  10

1  2  3  4  5  6  7  8  9  10

EP 0 384 566 A2

# FIG. 1C

# FIG. 1D

EP 0 384 566 A2

FIG. 2A

FIG. 2B

# FIG. 3

# FIG. 4A

```
pO9-1920   ------------------------------------------------------------
ATK-1      --------------------------------.---------------------------
pO1-12G    CCCGGGGGCTGGCCGCTCATCACTGGCTTAACTTCCTCCAAGCGGCATATCGCCTAGAAC   60
            R   G   L   A   A   H   H   W   L   N   F   L   Q   A   A   Y   R   L   E   P

pO9-1920   ------------------------------------------------------------
ATK-1      ----------------------------------A-------------------------
pO1-12G    CCGGTCCCTCCAGTTACGATTTCCACCAGTTGAAAAAAATTTCTTAAAATAGCTTTAGAAA   120
            G   P   S   S   Y   D   F   H   Q   L   K   K   F   L   K   I   A   L   E   T

pO9-1920   ------------------------------------------------------------
ATK-1      -------CTC---------------------------------------------------
pO1-12G    CACCGGTCTGGATCTGTCCCATTAACTACTCCCTCCTAGCCAGCCTACTCCCAAAAGGAT   180
             P   V   W   I   C   P   I   N   Y   S   L   L   A   S   L   L   P   K   G   Y

pO9-1920   ------------------------------------------------------------
ATK-1      ------------------------------------------------------------
pO1-12G    ACCCCGGCCGGGTGAATGAAATTTTACACATACTCATCCAAACCCAAGCCCAGATCCCGT   240
             P   G   R   V   N   E   I   L   H   I   L   I   Q   T   Q   A   Q   I   P   S

pO9-1920   ------------------------------------------------------------
ATK-1      ------------------------------------------------------------
pO1-12G    CCGGTCCCGCGCCACCGCCGCCGTCATCCCCCCACCCACGACCCCCCGGATTCTGATCCAC   300
             G   P   A   P   P   P   P   S   S   P   T   H   D   P   P   D   S   D   P   Q

pO9-1920   ------------------------------------------------------------
ATK-1      ------------------------------------C-------------T----
pO1-12G    AAATCCCCCCCTCCCTATGTTGAGCCTACGGCCCCCCAAGTTCTTCCAGTCATGCACCCAC   360
             I   P   P   P   Y   V   E   P   T   A   P   Q   V   L   P   V   M   H   P   H

pO9-1920   ----------------------------------------------T-------------
ATK-1      --------T-----T---------------------------------------------
pO1-12G    ATGGTGCCCCTCCCAACCATCGCCCATGGCAAATGAAAGACCTACAGGCCATTAAGCAAG   420
             G   A   P   P   N   H   R   P   W   Q   M   K   D   L   Q   A   I   K   Q   E

pO9-1920   ------------------------------------------------------------
ATK-1      ------------------------------------------------------------
pO1-12G    AAGTCTCCCAAGCAGCCCCTGGGAGCCCCCAGTTTATGCAGACCATCCGGCTTGCGGTGC   480
             V   S   Q   A   A   P   G   S   P   Q   F   M   Q   T   I   R   L   A   V   Q

pO9-1920   --------------------------------------------------
ATK-1      --------------------------------------------------
pO1-12G    AGCAGTTTGACCCCACTGCCAAAGACCTCCAAGACCTCCTGCAG   524
             Q   F   D   P   T   A   K   D   L   Q   D   L   L   Q
```

# FIG. 4B

```
ATK-1     ------------------------------------------------------------
PO1-12E   TCGACGCTCCAGGATATGACCCCATCTGGTTCCTTAATACCGAACCCAGCCAACTGCCTC    60
            D   A   P   G   Y   D   P   I   W   F   L   N   T   E   P   S   Q   L   P   P

ATK-1     ------------------------------------------------------------
PO1-12E   CCACCGCCCCTCCTCTACTCCCCCACTCTAACCTAGACCACATCCTCGAGCCCTCTATAC   120
            T   A   P   P   L   L   P   H   S   N   L   D   H   I   L   E   P   S   I   P

ATK-1     ---------------------A--------------------------------------
PO1-12E   CATGGAAATCAAAACTCCTGGCCCTTGTCCAGTTAACCCTACAAAGCACTAATTATACTT   180
            W   K   S   K   L   L   A   L   V   Q   L   T   L   Q   S   T   N   Y   T   C

ATK-1     --------------------------------C---------------C------------
PO1-12E   GCATTGTCTGTATCGATCGTGCCAGCCTATCCACTTGGCACGTTCTATACTCTCCCAACG   240
            I   V   C   I   D   R   A   S   L   S   T   W   H   V   L   Y   S   P   N   V

ATK-1     ------------------------------------------------------------
PO1-12E   TCTCTGTTCCATCCTCTTCTTCTACCCCCCTCCTTTACCCATCGTTAGCGCTTCCAGCCC   300
            S   V   P   S   S   S   T   P   L   L   Y   P   S   L   A   L   P   A   P

ATK-1     --------------------------------T---------------------------
PO1-12E   CCCACCTGACGTTACCATTTAACTGGACCCACCGCTTTGACCCCCAGATTCAAGCTATAG   360
            H   L   T   L   P   F   N   W   T   H   R   F   D   P   Q   I   Q   A   I   V

ATK-1     ------------------------------------------------------------
PO1-12E   TCTCCTCCCCCTGTCATAACTCCCTCATCCTGCCCCCCTTTTCCTTGTCACCTGTTCCCA   420
            S   S   P   C   H   N   S   L   I   L   P   P   F   S   L   S   P   V   P   T

ATK-1     ------------
PO1-12E   CCCTAGGATCC    431
            L   G   S
```

# FIG. 5

— 1300
— 1100
— 870

— 600

— 310
— 280
— 270
— 235
— 194
— 118
— 72

1    2    3 4 5 6 7 8 9  10